(19) Europäisches Patentamt
European Patent Office
Office européen des brevets

(11) Numéro de publication: **0 364 350 B1**

(12) **FASCICULE DE BREVET EUROPEEN**

(45) Date de publication de fascicule du brevet: **12.01.94**

(51) Int. Cl.[5]: **C07D 277/28**, A61K 31/495

(21) Numéro de dépôt: **89402781.2**

(22) Date de dépôt: **10.10.89**

(54) **Dérivés de méthyl-4[(phényl-4 pipérazinyl-1)-2 éthyl]-5 thiazole, leur procédé de préparation et les compositions pharmaceutiques en contenant.**

(30) Priorité: **11.10.88 FR 8813375**

(43) Date de publication de la demande:
**18.04.90 Bulletin 90/16**

(45) Mention de la délivrance du brevet:
**12.01.94 Bulletin 94/02**

(84) Etats contractants désignés:
**AT BE CH DE ES FR GB GR IT LI LU NL SE**

(56) Documents cités:
**FR-A- 2 594 335**

**CHEMICAL ABSTRACTS, vol. 85, no. 11, 13 septembre 1976, page 550, résumé no. 78164f, Columbus, Ohio, US.**

**CHEMICAL ABSTRACTS, vol. 100, no. 19, 7 mai 1984, page 529, résumé no. 156531t, Columbus, Ohio, US; M. BOGDAL et al.: "Thiazole derivatives. IV. Synthesis and pharmacological screening of 2-methyl-5-(2-N-substituted-aminoethyl)thiazoles".**

(73) Titulaire: **INSTITUT DE RECHERCHES CHIMIOUES ET BIOLOGIOUES APPLIOUEES (IRCEBA)**
**62, Grande-Rue**
**F-78490 Vicq(FR)**

(72) Inventeur: **Houziaux, Patrick**
**Chemin des Jonchères**
**Bazemont**
**F-78580 Maule(FR)**
Inventeur: **Lacolle, Jean-Yves**
**17, rue Charles de Gaulle**
**F-78860 Saint Nom La Breteche(FR)**
Inventeur: **Riffaud, Jean-Pierre**
**108, avenue des Etats Unis**
**F-78000 Versailles(FR)**
Inventeur: **Danree, Bernard**
**53, rue des Grands Champs**
**F-78300 Poissy(FR)**

(74) Mandataire: **Hubert, Philippe et al**
**Cabinet Beau de Loménie**
**158, rue de l'Université**
**F-75340 Paris Cédex 07 (FR)**

**EP 0 364 350 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne, en tant que produits nouveaux, les produits ayant la formule générale suivante :

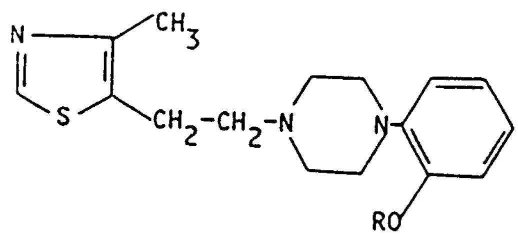

(I)

dans laquelle R est choisi parmi un atome d'hydrogène, un radical alkyle ayant de 2 à 7 atomes de carbone; un radical aralkyle, dans lequel la partie aryle consiste de préférence en phényle et dont la partie alkyle présente de 1 à 4 atomes de carbone, lesdits produits pouvant se présenter sous forme de sels pharmaceutiquement acceptables.

Dans la formule (I), les radicaux alkyle ou aralkyle peuvent être à chaîne droite ou ramifiée.

Un groupe alkyle ayant de 2 à 7 atomes de carbone est par exemple éthyle, propyle, isopropyle, butyle, tertiobutyle, pentyle, néopentyle, isopentyle, hexyle ou heptyle.

Un radical aralkyle est par exemple benzyle, éthylphényle, propylphényle, méthylnaphtyle, de préférence benzyle.

Les sels des produits de formule (I) sont obtenus, de façon connue, en mettant en contact un produit de formule (I) avec une quantité convenable d'acide pharmaceutiquement acceptable, minéral comme par exemple l'acide chlorhydrique ou l'acide sulfurique ou organique comme par exemple l'acide citrique, tartrique, maléique ou méthanesulfonique.

Le méthyl-4 [(phényl-4 pipérazinyl-1)-2 éthyl]-5 thiazole est décrit dans la demande de brevet français n° 2 594 335.

Des dérivés de ce composé ont également été décrits dans les publications Acta-Pol. Pharm. 40(2) page 159 et Pol. Pl 78 185,19 mai 1975. Cependant, les produits de formule (I) et leurs sels n'ont jamais été décrits dans la littérature.

La présente invention concerne également un procédé de préparation de produits de formule (I), ledit procédé consistant à faire réagir, sur un équivalent de méthyl-4 $\beta$-chloroéthyl-5 thiazole, soit deux équivalents de phényl-4 pipérazine substituée en position 2 sur le phényl par un groupe OR dans lequel R a la même signification qu'indiqué précédemment, soit un équivalent de gamma-picoline et un équivalent de phényl-4 pipérazine substituée, soit encore un équivalent de phényl-4 pipérazine substituée et du carbonate de sodium anhydre en excès, et à transformer éventuellement le produit ainsi obtenu en un de ses sels pharmaceutiquement acceptables. Ladite réaction est effectuée généralement à chaud et en milieu solvant.

La présente invention concerne également une composition pharmaceutique, destinée notamment au traitement des dysuries fonctionnelles liées à une hyperactivité du système sympathique $\alpha$-adrénergique, caractérisée en ce qu'elle contient, comme produit actif, au moins un produit de formule (I) ; ladite composition pouvant être utile notamment dans le domaine de l'urologie.

Sous cet aspect, l'invention vise également à couvrir un procédé de préparation de médicaments notamment utiles dans le domaine de l'urologie, caractérisé en ce que l'on incorpore, comme produit actif, au moins un produit de formule (I) dans un véhicule, excipient ou support pharmaceutiquement acceptable.

Le méthyl-4 [(phényl-4 pipérazinyl-1)-2 éthyl]-5 thiazole décrit dans la demande de brevet français 2 594 335 est également préconisé comme ingrédient actif dans des médicaments utiles dans le domaine de l'urologie.

Cependant, tous les composés conformes à la présente invention présentent, comme il sera démontré ci-après, des propriétés pharmacologiques très intéressantes et en particulier une dose efficace 50 ($DE_{50}$) très largement inférieure à celle de ce composé antérieurement connu.

Il a été découvert, de façon inattendue, que la famille particulière des dérivés du méthyl-4 [(phényl-4 pipérazinyl-1)-2 éthyl]-5 thiazole comportant sur le noyau phényle terminal un groupe hydroxy ou alcoxy en position 2 présentait des propriétés pharmacologiques très intéressantes et que la toxicité de ces dérivés était suffisamment faible pour permettre leur utilisation en thérapeutique.

Il en est également ainsi du dérivé comportant un groupe méthoxy en position 2 sur le noyau phényle. Ce composé, n'est pas revendiqué en tant que produit nouveau, puisqu'il a été décrit dans la publication précitée ACTA Pol. Pharm. 40 (2), page 159. Cependant, l'activité connue de ce dernier composé est tout à fait différente de l'activité mise en évidence par le demandeur.

C'est pourquoi, l'invention concerne également l'utilisation des composés de formule (I) précitée, dans laquelle le radical R a les significations indiquées précédemment, et peut être en outre un radical méthyle, pour la préparation de médicaments utiles pour le traitement des dysuries fonctionnelles liées à une hyperactivité du système sympathique, $\alpha$-adrénergique.

Sous un dernier aspect, la présente invention vise encore à couvrir un procédé de traitement des dysuries fonctionnelles liées à une hyperactivité du système sympathique $\alpha$-adrénergique, caractérisé en ce qu'il comprend l'administration en une quantité efficace d'au moins un composé de formule générale (I) dans laquelle le radical R a les significations indiquées précédemment et peut être en outre un radical méthyle.

Dans les exemples illustratifs suivants, le dosage par $AgNO_3$ est un test de pureté du produit.

Example 1

Synthèse du trichlorhydrate du méthyl-4 [(o-éthoxyphényl-4 pipérazinyl-1)-2 éthyle]-5 thiazole. Nom de code : B 1211. Produit de formule (1) avec R = éthyle sous forme de trichlorhydrate.

1) Préparation du méthyl-4 [(o-éthoxyphényl-4 pipérazinyl-1)-2 éthyl]-5 thiazole.

Dans un tricol de 100 ml muni d'un réfrigérant, d'une agitation pneumatique, d'un thermomètre et d'une ampoule à introduction, on introduit :

. 41,26 g (0,2 mol) o-éthoxyphényl-1 pipérazine
. 16,17 g (0,1 mol) méthyl-4 $\beta$-cloroéthyl-5 thiazole

Le milieu réactionnel est chauffé à 150 °C durant 1 h.

Après refroidissement à température ambiante, verser sur une solution de bicarbonate de sodium à 5 %.

La phase aqueuse obtenue est extraite par 3 x 150 ml de chlorure de méthylène.

Les phases organiques réuni es sont lavées par $H_2O$ saturée en chlorure de sodium. On les sèche sur sulfate de sodium. Après filtration, le solvant est chassé sous vide.

On obtient 31,49 g de produit brut (rendement brut = 95 %). Celui-ci est purifié par distillation fractionnée sous vide.

On isole 22,50 g d'un liquide brun (rendement après distillation = 67,9 %) ; $Eb_{0,4\ mmHg}$ = 185-190 °C ; une pureté C.P.V. > 99 %.

2) Préparation du trichlorhydrate :

16,57 g (0,05 mol) de ce produit sont dissous dans l'éther éthylique anhydre.

La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70 °C.

On obtient 19,24 g de cristaux beiges (rendement brut = 87,3 %).

Après recristallisation dans un mélange isopropanol/éthanol : 1/3, on isole 16,0 g de cristaux blancs (rendement après recristallisation = 72,6 %).

Lesdits cristaux présentent un point de fusion $F_{BK}$ de 212-213 °C, des spectres IR et RMN conformes à la structure proposée, une pureté C.P.V. > 99 %, un titre $AgNO_3$ de 100,2 %.

Exemple 2

Synthèse du bichlorhydrate du méthyl-4 [(o-hydroxyphényl-4 pipérazinyl-1)-2 éthyl]-5 thiazole. Nom de code : B 1302. Produit de formule (I) avec R = hydrogène sous forme de bichlorhydrate.

1) Préparation du méthyl-4 [(o-hydroxyphényl-4 pipérazinyl-1)-2 éthyl]-5 thiazole

Dans un tricol de 100 ml muni d'un réfrigérant, d'une agitation pneumatique, d'un thermomètre et d'une ampoule à introduction, on introduit :

. 35,65 g (0,2 mol) o-hydroxyphényl-1 pipérazine
. 16,17 g (0,1 mol) méthyl-4 β-chloroéthyl-5 thiazole

Le milieu réactionnel est chauffé à 150 ° C durant 1 h.

Après refroidissement à température ambiante et ajout de chlorure de méthylène, verser sur $H_2O$ saturée en chlorure de sodium.

Décanter la phase organique et extraire la phase aqueuse par 2 x 150 ml de chlorure de méthylène.

Les phases organiques réunies sont lavées par $H_2O$ saturée en chlorure de sodium. On les sèche sur sulfate de sodium. Après filtration, le solvant est chassé sous vide.

On obtient 27,61 g d'une huile brune (rendement brut = 91 %) ayant une pureté C.P.V. = 97 %.

2) Préparation du bichlorhydrate :

15,17 g (0,05 mol) de ce produit brut sont dissous dans 430 ml d'éther éthylique anhydre.

La solution est saturée par un courant d'acide chlorhydrique gazeux sec sur un bain de glace.

Les cristaux formés sont filtrés sur verre fritté, lavés par de l'éther éthylique anhydre, puis séchés sous vide sur potasse à 70 ° C.

On obtient 18,06 g de cristaux beiges (rendement brut = 96 %).

Après deux recristallisations dans un mélange isopropanol/méthanol : 1/1, on isole 4,80 g de cristaux beige clair (rendement après recristallisations = 25,5 %).

Lesdits cristaux présentent un point de fusion $F_{BK}$ de 250-255 ° C, des spectres IR et RMN conformes à la structure proposée, un titre $AgNO_3$ de 100,5 %.

Exemple 3

Synthèse de monochlorhydrate du méthyl-4 [(o-butoxyphényl-4 pipérazinyl-1)-2 éthyl]-5 thiazole. Nom de code : B 1341. Produit de formule (I) avec R = butyle sous forme de monochlorhydrate.

1) Préparation du méthyl-4 [(o-butoxyphényl-4 pipérazinyl-1)-2 éthyl]-5 thiazole

Dans un tricol de 100 ml muni d'un réfrigérant, d'une agitation pneumatique, d'un thermomètre et d'une ampoule à introduction, on introduit :

. 46,87 g (0,2 mol) o-butoxyphényl-1 pipérazine.
. 16,17 g (0,1 mol) méthyl-4 β-chloroéthyl-5 thiazole.

Le milieu réactionnel est chauffé à 150 ° C durant 1 h.

Après refroidissement à température ambiante et ajout d'éther éthylique, verser sur $H_2O$. Décanter la phase organique, et extraire la phase aqueuse restante par deux fois 150 ml d'éther éthylique. Les phases organiques réunies sont lavées par de l'eau saturée en chlorure de sodium. On les sèche sur sulfate de sodium. Après filtration, le solvant est chassé sous vide.

On obtient 33,76 g d'une huile brune (rendement brut = 93,9 %) qui est purifiée par distillation fractionnée sous vide.

On isole 24,16 g d'un liquide orangé (rendement après distillation = 67,2 %) ; $Eb_{0,1\ mm\ Hg}$ = 180-185 ° C ; une pureté C.P.V. = 98,8 %).

2) Préparation du monochlorhydrate :

17,97 g (0,05 mol) de ce produit sont dissous dans l'éthanol absolu.

Après refroidissement dans un bain de glace, on additionne 43,5 ml d'une solution éthanolique d'HCl (titrant 1,15 mol HCl par litre).

Après avoir laissé sous agitation durant 30 min, concentrer sous vide à sec, reprendre le résidu par de l'éther anhydre.

Les cristaux formés sont filtrés sur verre fritté, lavés par de l'éther éthylique anhydre, puis séchés sous vide sur potasse à 70 ° C.

On obtient 19,16 g de cristaux beiges (rendement brut = 96,8 %).

Après recristallisation dans l'isopropanol, on isole 16,06 g de cristaux blancs (rendement après recristallisation = 81,1 %).

Lesdits cristaux présentent un point de fusion $F_{BK}$ de 180-185 ° C, des spectres IR et RMN conformes à la structure proposée, un titre $AgNO_3$ de 98,6 %, une pureté C.P.V. > 99,5 %.

Exemple 4

Synthèse du monochlorhydrate du méhthyl-4 [(o-pentoxyphényl-4 pipérazinyl-1)-2 éthyl]-5 thiazole. Nom de code : B 1357.
Produit de formule (I) avec R = pentyle sous forme de monochlorhydrate.

1) Préparation du méthyl-4 (o-pentoxyphényl-4 pipérazinyl-1)-2 éthyl]-5 thiazole.

Dans un tricol de 100 ml muni d'un réfrigérant, d'une agitation pneumatique, d'un thermomètre, on introduit :

. 27,32 g (0,11 mol) o-pentoxyphényl-1 pipérazine
. 8,89 g (0,055 mol) méthyl-4 $\beta$-chloroéthyl-5 thiazole.

Le milieu réactionnel est chauffé à 150 °C durant 1 h 30 min.

Après refroidissement à température ambiante et ajout de 200 ml d'éther éthylique, verser sur 200 ml $H_2O$. Décanter la phase organique et extraire la phase aqueuse restante par deux fois 100 ml d'éther éthylique. Les phases organiques réunies sont lavées par de l'eau saturée en chlorure de sodium. On les sèche sur sulfate de sodium. Après filtration, le solvant est chassé sous vide.

On obtient 19,04 g d'une huile brune (rendement brut = 92,6 %) qui est purifiée par distillation fractionnée sous vide.

On isole 13,25 g d'un liquide orangé (rendement après distillation = 64,5 %).

$Eb_{0,1\ mmHg}$ = 190-193 °C; une pureté C.P.V = 99,9 %; un titre perchlorique = 100,6 %.

2) Préparation du monochlorhydrate.

12,44 g (0,0333 mol) de ce produit sont dissous dans 150 ml d'éthanol absolu. Refroidir la solution dans un bain de glace, on additionne 23,1 ml d'une solution éthanolique d'HCl (titrant 1,44 mol HCl par litre).

Après avoir laissé sous agitation durant 5 min, concentrer sous vide à sec, reprendre le résidu par de l'éther éthylique anhydre.

Les cristaux formés sont filtrés sur verre fritté, lavés par de l'éther éthylique, puis séchés sous vide sur potasse à 70 °C.

On obtient 13,38 g de cristaux beiges (rendement brut : 98 %).

Après recristallisation dans un mélange acétate d'éthyleisopropanol (20/1), on isole 10,35 g de cristaux blancs (rendement après recristallisation = 75,8 %).

Lesdits cristaux présentent un point de fusion $F_{BK}$ de 156-158 °C; des spectres I.R. et R.M.N. conformes à la structure proposée, un titre $AgNO_3$ de 97,7 %, une pureté C.P.V. = 99,9 %.

Exemple 5

Synthèse du monochlorhydrate du méthyl-4 [(o-isopropoxyphényl-4 pipérazinyl-1)-2 éthyl]-5 thiazole. Nom de code : B 1398.
Produit de formule (I) avec R = isopropyle sous forme de monochlorhydrate.

1) Préparation du méthyl-4 [(o-isopropoxyphényl-4 pipérazinyl-1)-2 éthyl]-5 thiazole.

Dans un tricol de 100 ml muni d'un réfrigérant, d'une agitation pneumatique, d'un thermomètre, on introduit :

. 29,41 g (0,133 mol) o-isopropoxyphényl-1 pipérazine
. 10 g (0,062 mol) méthyl-4 $\beta$-chloroéthyl-5 thiazole.

Le milieu réactionnel est chauffé à 150 °C durant 1 h.

Après refroidissement à température ambiante et ajout de chlorure de méthylène, verser sur une solution de $NaHCO_3$ 10 %. Décanter la phase organique et extraire la phase aqueuse restante par deux fois 100 ml $CH_2Cl_2$. Les phases organiques réunies sont lavées jusqu'à neutralité par de l'eau saturée en chlorure de sodium. On les sèche sur sulfate de sodium. Après filtration, le solvant est chassé sous vide.

On obtient une huile brute contenant l'excès d'o-isopropoxyphényl-1 pipérazine ayant servi d'accepteur d'HCl. La purification est effectuée par distillation fractionnée sous vide.

On isole 17,2 g d'un liquide orangé (rendement après distillation = 80,5 %).

$Eb_{0,15\ mmHg}$ = 225-230 °C; une pureté C.P.V. = 99 %.

2) Préparation du monochlorhydrate.

17,2 g (0,05 mol) de ce produit sont dissous dans l'éthanol absolu.

Après refroidissement dans un bain de glace, on additionne 37 ml d'une solution éthanolique d'HCl (titrant 1,35 mol HCl par litre).

Après avoir laissé sous agitation durant 5 min, filtrer le précipité ainsi formé sur verre fritté, le laver par de l'éther éthylique anhydre, puis le sécher sous vide sur potasse à 70 ° C.

On obtient 13,9 g de cristaux blancs (rendement brut : 72,7 %).

Après recristallisation dans un mélange acétate d'éthyle/éthanol (3/1), on isole 11,6 g de cristaux blancs (rendement après recristallisation : 60,6 %).

Lesdits cristaux présentent un point de fusion $F_{BK}$ de 196-200 ° C, des spectres I.R. et R.M.N. conformes à la structure proposée, un titre $AgNO_3$ de 98 %, une pureté C.P.V. = 99,9 %.

Exemple 6

Synthèse du trichlorhydrate du méthyl-4 (o-méthoxyphényl-4 pipérazinyl-1)-2 éthyl]-5 thiazole. Nom de code B 1 433.

Produit de formule (I) avec R = méthyle.

1) Préparation du méthyl-4 [(o-méthoxyphényl-4 pipérazinyl-1)-2 éthyl]-5 thiazole

Dans un tricol de 500 ml muni d'un réfrigérant, d'une agitation pneumatique, d'un thermomètre, d'une ampoule à introduction, on introduit :

. 210 ml butanol-1

. 10,66 g (0,0554 mol) o-méthoxyphényl-1 pipérazine

. par portions 8,81 g (0,0831 mol) $Na_2CO_3$ anhydre.

On additionne goutte à goutte 8,96 g (0,0554 mol) de méthyl-4 $\beta$-chloroéthyl-5 thiazole.

Le milieu réactionnel est chauffé au reflux durant 50 h.

Après filtration à chaud du milieu réactionnel et lavage des sels insolubles par de l'éthanol, le filtrat est concentré à sec sous vide.

On obtient 15,2 g d'une huile brune (rendement brut : 86,4 %) ayant une pureté C.P.V. = 94,2 %.

2) Préparation du trichlorhydrate

14,5 g (0,0457 mol) de ce produit sont dissous dans 80 ml d'éther éthylique anhydre.

Après refroidissement dans un bain de glace, par barbotage, on sature la solution en HCl gazeux.

On laisse 15 min sous agitation. Les cristaux formés sont filtrés sur verre fritté, lavés par de l'éther éthylique anhydre, puis séchés sous vide sur potasse à 70 ° C.

On obtient 17,06 g de cristaux (rendement brut : 87,5 %).

Après recristallisation dans 330 ml d'éthanol, on isole 9 g de cristaux blancs (rendement après recristallisation = 46,2 %).

Lesdits cristaux présentent un point de fusion $F_{BK}$ de 210-212 ° C, des spectres I.R. et R.M.N. conformes à la structure proposée, un titre $AgNO_3$ de 96,5 %, une pureté C.P.V. = 99,9 %.

Exemples 7 à 11

En suivant des processus expérimentaux semblables à ceux qui viennent d'être décrits, et que l'homme de métier retrouvera facilement, on a réalisé les composés de formule (I), dans laquelle R représente respectivement un radical propyle (exemple 7), isobutyle (exemple 8), isopentyle (exemple 9), néopentyle (exemple 10) et benzyle (exemple 11).

On a résumé, dans le tableau récapitulatif n° 1 les formules et les propriétés physiques des produits synthétisés dans les exemples 1 à 11.

La toxicité et les propriétés pharmacologiques des produits de formule I ont été testées ; les résultats obtenus sont décrits ci-après.

1) Toxicité aiguë chez la souris

Principe de la mesure

Les produits ont été administrés par voie orale à dose unique chez la Souris mâle d'un poids moyen de 22 g. La mortalité a été enregistrée après une période d'observation de 14 jours.

Les résultats sont exprimés sous forme de dose létale 50 ($D.L_{50}$) : dose théorique en $mg.kg^{-1}$, p.o, provoquant la mort de 50 % des animaux.

Résultats

Ils sont rapportés dans le tableau 2.

Pratiquement toutes les molécules ont une $D.L_{50}$ supérieure à $1g.kg^{-1}$. Ce sont donc des produits peu toxiques après une seule administration.

2) Détermination de l'activité alpha-bloquante sur canal déférent isolé de Rat

a) Principe de la mesure

La stimulation des récepteurs alpha-adrénergiques postsynaptiques par la noradrénaline provoque la contraction du canal déférent isolé.

On détermine la concentration de produit en présence de laquelle il faut multiplier par deux la concentration de noradrénaline pour obtenir le même effet qu'en l'absence dudit produit.

Le logarithme changé de signe de cette concentration constitue la $pA_2$ des produits.

b) Résultats obtenus

Les valeurs des $pA_2$ rapportées dans le tableau 3 montrent que les produits se comportent comme des antagonistes compétitifs de la noradrénaline au niveau des récepteurs alpha-adrénergiques.

Leur activité alpha-bloquante est importante puisqu'elle apparaît pour des concentrations faibles, entre $2.10^{-7}$ M et $10^{-8}$ M.

3) Détermination de l'activité adrénolytique "in vivo" chez le Rat

a) Principe de la mesure

L'injection intraveineuse de noradrénaline (0,4 $mg.kg^{-1}$) chez le Rat vigile provoque la mort de 100 % des animaux. L'administration préalable d'une subtance à propriété alpha-bloquante permet de réduire cette toxicité.

Les résultats sont exprimés sous forme de dose efficace 50 ($D.E_{50}$), dose en $mg.kg^{-1}$, protégeant 50 % des animaux.

b) Résultats obtenus

Les $D.E_{50}$ correspondant aux produits des exemples 1 à 11 sont portées dans le tableau 4.

Les produits sont pour la plupart actifs à des doses relativement faibles. Leur activité adrénolytique, chez l'animal entier est donc très importante, confirmant ainsi les effets mis en évidence in vitro.

Il est à noter que tous ces composés présentent une $DE_{50}$ très largement inférieure (gain d'environ 40 à environ 90 %) à celle du méthyl-4 [(phényl-4 pipérazinyl-1)-2 éthyl]-5 thiazole (qui est de 8,7 $mg.kg^{-1}$ dans les mêmes conditions).

Ces résultats démontrent l'intérêt de ces composés et leur nette supériorité vis-à-vis du composé le plus proche de l'état de la technique.

4) Détermination de l'action inhibitrice des composés sur l'augmentation de pression uretrale induite chez le lapin par la noradrenaline par voie I.V.

a) Principe de la mesure

La pression urétrale est mesurée par cathétérisme de l'urètre prostatique chez le Lapin mâle anesthésié. L'augmentation de la pression urétrale est induite par une injection intraveineuse de noradrénaline. Cette hyperpression peut être inhibée par l'administration préalable de molécules à potentiel alphabloquant.

Seuls les composés : B 1211, B 1302, B 1341, B 1357, B 1398 et B 1433 ont été essayés sur ce modèle

b) Résultats obtenus

Ils sont présentés dans le tableau 5, sous forme de dose inhibitrice 50 % ($D.I_{50}$) définie comme étant la dose de produit qui admnistrée en i.v inhibe de 50 % l'hyperpression urétrale induite par la noradrénaline.

Les composés testés sont particulièrement efficaces avec des doses actives inférieures à 1 $mg.kg^{-1}$.

Le B 1398 présente une efficacité remarquable.

Ces données laissent présager une bonne activité thérapeutique de ces molécules dans les dysuries fonctionnelles liées à une hyperactivité du système sympathique alpha-adrénergique.

Les produits peuvent être administrés par voie générale (parentérale, orale, rectale) ou par voie topique.

Les compositions pharmaceutiques contenant, à titre d'ingrédient actif, au moins un produit selon l'invention en combinaison avec un véhicule pharmaceutiquement acceptable peuvent être solides ou liquides et se présenter, par exemple, sous forme de préparations injectables, de comprimés, gélules, granulés. La posologie peut varier dans de larges proportions, en particulier suivant le type et la gravité de l'affection à traiter et suivant le mode d'administration.

Le plus souvent, chez l'adulte, par voie parentérale, elle est comprise entre 0,05 et 0,5 g par jour; par voie orale, elle est comprise entre à 0,1 et 4 g par jour.

TABLEAU 1

**FORMULE GENERALE :**

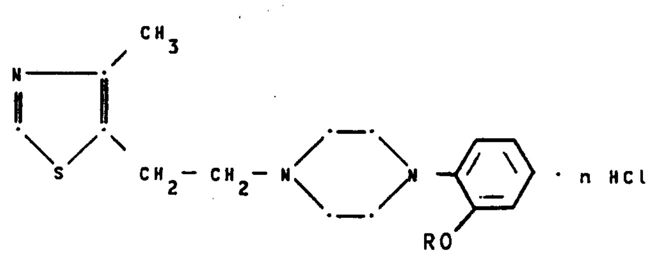

| Exemple | R | n | Code B | Formule brute | P.M. | $F_{BK}$(°C) |
|---|---|---|---|---|---|---|
| 1 | $-C_2H_5$ | 3 | 1211 | $C_{18}H_{28}Cl_3N_3OS$ | 440,85 | 212-213 |
| 2 | -H | 2 | 1302 | $C_{16}H_{23}Cl_2N_3OS$ | 376,35 | 250-255 |
| 3 | $-(CH_2)_3-CH_3$ | 1 | 1341 | $C_{20}H_{30}ClN_3OS$ | 396,0 | 180-185 |
| 4 | $-(CH_2)_4-CH_3$ | 1 | 1357 | $C_{21}H_{32}ClN_3OS$ | 410,04 | 156-158 |
| 7 | $-(CH_2)_2-CH_3$ | 1 | 1359 | $C_{19}H_{28}ClN_3OS$ | 381,96 | 189-190 |
| 5 | 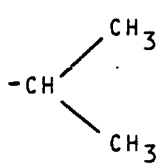 | 1 | 1398 | $C_{19}H_{28}ClN_3OS$ | 381,96 | 196-200 |

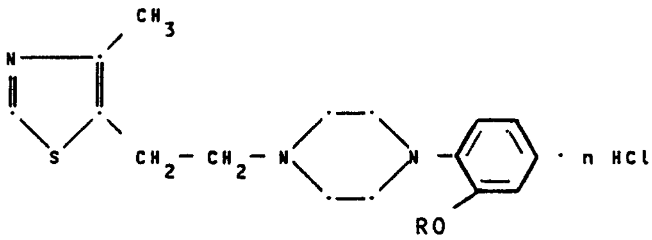

| Exemple | R | n | Code B | Formule brute | P.M. | $F_{BK}$(°C) |
|---|---|---|---|---|---|---|
| 8 | 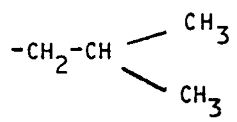 | 1 | 1422 | $C_{20}H_{30}Cl\ N_3\ O\ S$ | 395,95 | 188-189 |
| 9 | 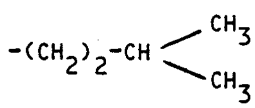 | 1 | 1423 | $C_{21}H_{32}Cl\ N_3\ O\ S$ | 410,04 | 160-162 |
| 10 | 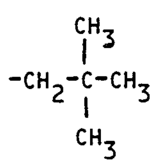 | 1 | 1424 | $C_{21}H_{32}Cl\ N_3\ O\ S$ | 410,04 | 200-202 |
| 11 | 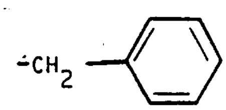 | 2 | 1425 | $C_{23}H_{29}Cl_2\ N_3\ O\ S$ | 466,45 | 175-180 |
| 6 | $-CH_3$ | 3 | 1433 | $C_{17}H_{26}Cl_3\ N_3\ O\ S$ | 426,82 | 210-212 |

Tableau 2

TOXICITE AIGUE DES COMPOSES CHEZ LA SOURIS APRES ADMINISTRATION UNIQUE PAR VOIE ORALE

($D.L_{50}$ = Dose létale 50 p. cent)

| Code B | 1211 | 1302 | 1341 | 1357 | 1359 | 1398 | 1422 | 1423 | 1424 | 1425 | 1433 |
|---|---|---|---|---|---|---|---|---|---|---|---|
| $D.L_{50}$ $mg.kg^{-1}p.o$ | >1000 | 490 | >1000 | >1000 | >1000 | >1000 | >1000 | >1000 | >1000 | >1000 | >1000 |

Tableau 3

ACTIVITE ALPHA-BLOQUANTE DES COMPOSES VIS A VIS DE LA NORADRENALINE SUR CANAL DEFERENT ISOLE DE RAT

| Code B | 1211 | 1302 | 1341 | 1357 | 1359 | 1398 | 1422 | 1423 | 1424 | 1425 | 1433 |
|---|---|---|---|---|---|---|---|---|---|---|---|
| $pA_2$ | 7.58 | 6.90 | 8.0 | 7.77 | 7.24 | 7.77 | 7.44 | 7.34 | 6.7 | 7.20 | 7.84 |

Tableau 4

ACTIVITE ADRENOLYTIQUE DES COMPOSES VIS A VIS DE L'EFFET LETAL DE LA NORADRENALINE CHEZ LE RAT PAR VOIE ORALE

($D.E_{50}$ = Dose efficace 50 p.cent)

| Code B | 1211 | 1302 | 1341 | 1357 | 1359 | 1398 | 1422 | 1423 | 1424 | 1425 | 1433 |
|---|---|---|---|---|---|---|---|---|---|---|---|
| $D.E_{50}$ $mg.kg^{-1}$ p.o | 2 | 4 | 1 | 1.80 | 1.93 | 0.44 | 1.60 | 2.35 | 2.04 | 5 | 2.66 |

Tableau 5

ACTION INHIBITRICE DES COMPOSES SUR L'AUGMENTATION DE PRESSION URETRALE INDUITE CHEZ LE LAPIN PAR LA NORADRENALINE PAR VOIE I.V. ($D.I_{50}$ : Dose inhibitrice 50 p.cent)

| Code B | 1211 | 1302 | 1341 | 1357 | 1359 | 1398 | 1422 | 1423 | 1424 | 1425 | 1433 |
|---|---|---|---|---|---|---|---|---|---|---|---|
| $D.I_{50}$ $mg.kg^{-1}$ i.v | 0.16 | 0.44 | 0.39 | 0.46 | - | 0.09 | - | - | - | - | 0.3 |

## Revendications

**1.** Dérivés du méthyl-4 [(phényl-4 pipérazinyl-1)-2 éthyl]-5 thiazole, caractérisé en ce qu'ils répondent à la formule générale :

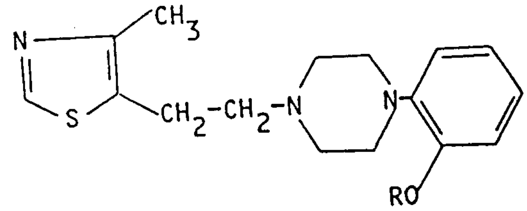

(I)

dans laquelle R est choisi parmi un atome d'hydrogène, un radical alkyle ayant de 2 à 7 atomes de carbone, un radical aralkyle, dans lequel la partie aryle consiste de préférence en phényle et dont la partie alkyle présente de 1 à 4 atomes de carbone, ainsi que les sels desdits produits de formule (I) pharmaceutiquement acceptables.

**2.** Dérivé selon la revendication 1, caractérisé en ce que R est choisi parmi un atome d'hydrogène ou un radical éthyle, isopropyle, butyle, pentyle.

**3.** Procédé de préparation des dérivés selon la revendications 1, caractérisé en ce qu'il consiste à faire réagir sur un équivalent de méthyl-4 $\beta$-chloroéthyl-5 thiazole, soit deux équivalents de phényl-4 pipérazine substituée en position 2 sur le phényl par un groupe OR dans lequel R a la même signification qu'indiqué précédemment, soit un équivalent de gamma-picoline et un équivalent de phényl-4 pipérazine substituée, soit encore un équivalent de phényl-4 pipérazine substituée et du carbonate de sodium anhydre en excès, et à transformer éventuellement le produit ainsi obtenu en un de ses sels pharmaceutiquement acceptable. Ladite réaction étant effectuée à chaud dans un milieu solvant.

**4.** Composition pharmaceutique, caractérisée en ce qu'elle contient, à titre d'ingrédient actif, au moins un dérivé selon la revendication 1 ou 2, en association avec un véhicule, excipient ou support pharmaceutiquement acceptable.

**5.** Composition pharmaceutique destinée au traitement des dysuries fonctionnelles liées à une hyperactivité du système sympathique $\alpha$-adrénergique, caractérisée en ce qu'elle contient à titre d'ingrédient actif, au moins un dérivé selon la revendication 1 ou 2, en association avec un véhicule, excipient ou support pharmaceutiquement acceptable.

**6.** Procédé pour la préparation de compositions pharmaceutiques, caractérisé en ce qu'il consiste à mélanger à des excipients pharmaceutiquement acceptables une dose efficace d'un dérivé selon la revendication 1 ou 2.

**7.** Utilisation des dérivés selon la revendication 1, pour la préparation de médicaments destinés au traitement des dysuries fonctionnelles liées à une hyperactivité du système sympathique $\alpha$-adrénergique.

## Claims

**1.** 4-methyl-5-[2-(4-phenylpiperazin-1-yl)ethyl]thiazole derivatives, characterized in that they have the general formula

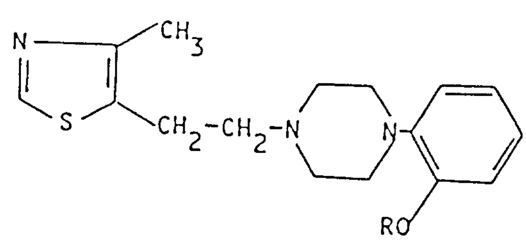

(I)

in which R is selected from a hydrogen atom, an alkyl radical having from 2 to 7 carbon atoms and an aralkyl radical in which the aryl moiety preferably consists of phenyl and of which the alkyl moiety has from 1 to 4 carbon atoms, as well as the pharmaceutically acceptable salts of said products of formula (I).

**2.** Derivative according to claim 1, characterized in that R is selected from a hydrogen atom or an ethyl, isopropyl, butyl or pentyl radical.

**3.** Method of preparing derivatives according to claim 1, characterized in that it consists in reacting, with one equivalent of 4-methyl-5-$\beta$-chloroethylthiazole, either two equivalents of 4-phenylpiperazine substituted in the 2-position on the phenyl by a group OR, in which R is as defined above, or one equivalent of gamma-picoline and one equivalent of substituted 4-phenylpiperazine, or else one equivalent of substituted 4-phenylpiperazine and an excess of anhydrous sodium carbonate, and, if desired, in converting the resulting product to one of its pharmaceutically acceptable salts. Said reaction being generally carried out with heating and in a solvent medium.

**4.** Pharmaceutical composition, characterized in that it contains at least one derivative according to claim 1 or 2 as the active ingredient, in association with a pharmaceutically acceptable vehicle, excipient or carrier.

**5.** Pharmaceutical composition intended for the treatment of functional dysuria associated with hyperactivity of the $\alpha$-adrenergic sympathetic nervous system, characterized in that it contains at least one derivative according to claim 1 or 2 as the active ingredient, in association with a pharmaceutically acceptable vehicle, excipient or carrier.

**6.** Method of preparing pharmaceutical compositions, characterized in that it consists in mixing an effective amount of a derivative according to claim 1 or 2 with pharmaceutically acceptable excipients.

**7.** Use of the derivatives according to claim 1, for the preparation of drugs intended for the treatment of functional dysuria associated with hyperactivity of the $\alpha$-adrenergic sympathetic nervous system.

**Patentansprüche**

**1.** Derivate des 4-Methyl-5-[2-(4-phenylpiperazin-1-yl)ethyl]-thiazols,die gekennzeichnet sind durch die allgemeine Formel I

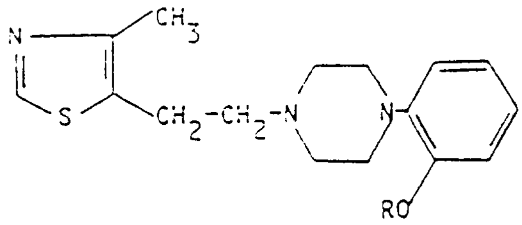

(I),

in der R unter Wasserstoff, $C_{2-7}$- Alkyl und Aralkyl, bei dem der Arylteil vorzugsweise Phenyl ist und dessen Alkylteil 1 bis 4 Kohlenstoffatome aufweist, ausgewählt ist,
sowie die pharmazeutisch akzeptablen Salze der Verbindungen der Formel I.

**2.** Derivate nach Anspruch 1, dadurch gekennzeichnet, daß R unter Wasserstoff und Ethyl, Isopropyl, Butyl und Pentyl ausgewählt ist.

**3.** Verfahren zur Herstellung der Derivate nach Anspruch 1, gekennzeichnet durch Umsetzung eines Äquivalents 4-Methyl-5-$\beta$-chlorethylthiazol entweder mit zwei Äquivalenten 4-Phenylpiperazin, das in 2-Stellung am Phenylkern mit einer Gruppe OR substituiert ist, wobei R die oben angegebene Bedeutung hat, oder mit einem Äquivalent $\gamma$-Picolin und einem Äquivalent substituiertem 4-Phenylpiperazin oder mit einem Äquivalent substituiertem 4-Phenylpiperazin und überschüssigem wasserfreiem Natriumcarbonat sowie ggfs. Umwandlung des so erhaltenen Produkts in eines seiner pharmazeutisch akzeptablen Salze, wobei die Umsetzung in der Wärme in einem Lösungsmittelmedium durchgeführt wird.

**4.** Pharmazeutische Zusammensetzung, dadurch gekennzeichnet, daß sie als Wirkstoff mindestens ein Derivat nach Anspruch 1 oder 2 neben einem pharmazeutisch akzeptablen Excipiens oder flüssigen oder festen Träger enthält.

**5.** Pharmazeutische Zusammensetzung, die zur Behandlung von funktionellen Dysurien bestimmt ist, die durch eine Hyperaktivität des α-adrenergen sympathischen Systems bedingt sind, dadurch gekennzeichnet, daß sie als Wirkstoff mindestens ein Derivat nach Anspruch 1 oder 2 neben einem pharmazeutisch akzeptablen Excipiens oder flüssigen oder festen Träger enthält.

**6.** Verfahren zur Herstellung pharmazeutischer Zusammensetzungen, gekennzeichnet durch Mischen einer wirksamen Dosis eines Derivats nach Anspruch 1 oder 2 mit pharmazeutisch akzeptablen Excipientien.

**7.** Verwendung der Derivate nach Anspruch 1 zur Herstellung von Arzneimitteln zur Behandlung von funktionellen Dysurien, die durch eine Hyperaktivität des α-adrenergen sympathischen Systems bedingt sind.